# EUROPÄISCHE PATENTSCHRIFT

(11) **EP 2 995 282 B1**
(45) Veröffentlichungstag und Bekanntmachung des Hinweises auf die Patenterteilung: **05.07.2017**
(21) Anmeldenummer: 15190138.6
(22) Anmeldetag: 21.07.2003
(51) Int. Cl.: A61F 2/91, A61B 17/221, A61F 2/92, A61B 17/12, A61F 2/915, A61F 2/95

(54) **VORGEFORMTER STENT**
PREFORMED STENT
STENT PRÉFORMÉ

(30) Priorität: 19.07.2002 DE 10233085
(43) Veröffentlichungstag der Anmeldung: 16.03.2016
(62) Teilanmeldung aus: 03765067.8
(73) Patentinhaber: Dendron GmbH, 44799 Bochum (DE)
(72) Erfinder: Henkes, Hans, 44797 Bochum (DE); Flesser, Achim, 40822 Mettmann (DE); Kontek, Ronald, 44651 Herne (DE); Speder, Jürgen, 44807 Bochum (DE); Bodenburg, Ralph, 44801 Bochum (DE)
(74) Vertreter: Hoffmann Eitle

(56) Entgegenhaltungen:
- EP-A2- 0 201 466
- WO-A-01/32099
- WO-A2-02/054980
- US-A- 5 984 929

## Beschreibung

Die Erfindung betrifft ein medizinisches Implantat, welches zur Einnahme einer übergeordneten Struktur am Implantationsort vorgeformt ist, während der Einführung jedoch in einer volumenreduzierten Form vorliegt.

Die Erfindung betrifft weiterhin die Verwendung dieses Implantates als Neurostent, seine Kombination mit einem Führungsdraht sowie ein System zur Verwendung derartiger Implantate bei der Behandlung von Aneurysmen oder anderer vaskulärer Fehlbildungen.

Es ist bekannt, Gefäßverengungen (Stenosen) durch den Einsatz von Stents (Gefäßendoprothesen, Gefäßstützen) zu behandeln, welche in den stenotischen Bereich eingeschoben werden und dort durch ihre Eigensteifigkeit das Gefäßlumen offen halten. Auch ist bekannt, solche Stents zum Verschluß von Gefäßwandaussackungen (Aneurysmen) oder Fisteln einzusetzen.

Herkömmlicherweise werden dabei ballondilatierbare Stents eingesetzt. Diese werden zur Einbringung in nicht-dilatiertem Zustand auf einen nichtexpandierten Ballon aufgekrimpt, über ein Kathetersystem an die zu behandelnde Stelle geführt und dort durch Expansion des Ballons dilatiert und so im Gefäß verankert. Da zur Einführung ballondilatierbarer Stents keine aufwendigen Stütz- und Überhüllen notwendig sind, können diese auch in sehr feine Gefäße eingeführt werden. Problematisch ist jedoch, daß sie aufgrund ihrer plastischen Verformbarkeit durch Druckeinwirkung von außen einfach zusammengedrückt werden können. Ein weiterer Nachteil besteht darin, daß sie zur Verankerung mittels Beaufschlagung mit hohem Druck zunächst über den Umfang ausgedehnt werden müssen, den sie letztlich einnehmen. Diese Aufweitung über den notwendigen Umfang birgt die Gefahr einer Gefäßverletzung, was die Bildung von Thromben nach sich ziehen kann.

Ein weiterer Nachteil dieser herkömmlichen ballondilatierbaren Stents beruht darauf, daß sie aufgrund ihrer Struktur nicht einfach durch einen gelegten Mikrokatheter hindurch an den Bestimmungsort geschoben werden können, sondern in einem speziell dafür ausgelegten Mikrokatheter in dessem distalen Bereich angeordnet werden müssen, um über einen sogenannten Pusher an den Implantationsort befördert zu werden. Dies macht die dazu benötigte Kathetertechnik aufwendig und schwer handhabbar. Ein weiteres Problem ist, daß ein einmal ausgebrachter Stent, wenn überhaupt, nur sehr schwer relokalisiert oder zurückgeholt werden kann. Ein einmal dilatierter Stent kann, wenn er falsch platziert wurde, in der Regel nicht mehr in seiner Position verändert oder wieder entfernt werden.

Es ist ferner bekannt, selbstexpandierende Stents einzusetzen, die aus Formgedächtnismaterialien gefertigt sind. Diese besitzen eine geflechtartige Struktur und werden zunächst in kollabiertem Zustand durch einen Katheter an den Zielort geführt, wo sie sich in Folge der Temperaturveränderung (thermischer Formgedächtniseffekt) oder durch den Wegfall des durch den Katheter ausgeübten mechanischen Zwanges (Superelastizität) aufweiten. Solche Stents weisen ebenfalls den Nachteil auf, daß die zur Einführung notwendigen Vorrichtungen relativ aufwendig und platzintensiv sind. So ist bei den bekannten superelastisch expandierbaren Stents stets eine Stütz- und Überhülle nötig, was einen relativ großen Katheterumfang bedingt und die Einbringung solcher Stents durch einen bereits gelegten Katheter ebenfalls ausschließt.

Für den Einsatz in besonders kleinlumigen intrakraniellen Gefäßen ist es ferner bekannt, Stents aus Formgedächtnismaterial einzusetzen, die zunächst als lang gestrecktes Filament vorliegen und erst bei Austritt durch den Katheter durch die Temperaturveränderung oder den Wegfall des zuvor aus dem Katheter ausgeübten Zwangs die röhrenförmige Struktur eines Stents annehmen.

Aus der DE 197 03 482 A1 ist es bekannt, zur Behandlung von Aneurysmen und dergleichen einen Stent aus zwei lang gestreckten Filamenten einzusetzen, welche durch den mechanischen Zwang des Stranges spannungsinduziert in der lang gestreckten Form gehalten werden und nach Wegfall dieses Zwanges, bei Herausschieben aus dem Katheter, die eigentliche Stent-Form einnehmen. Hierdurch wurde erstmals der Einsatz von Stents mit Formgedächtniseigenschaften auch in sehr kleinlumigen Gefäßen in Folge der intrakraniellen und zerebralen Gefäßäste möglich. Aus der US 5,984,929 ist ein weiterer Stent zur Behandlung von Aneurysmen bekannt, der alle technischen Merkmale aus dem Oberbegriff des Anspruchs 1, aufweist. Diese Stents, die für gewisse Einsatzzwecke gut geeignet sind, weisen aber eine Reihe von Nachteilen auf, darunter ihre relativ schwere Verschiebbarkeit im und die fehlende Rückführmöglichkeit in den Katheter, letzteres bei Fehlplatzierung. Auch ist der Stent, aufgrund seiner sehr filigranen Struktur, wenig geeignet, Aussackungen und Fisteln in den Gefäßen so abzudecken, daß darin platzierte Okklusionsmitkel zurückgehalten werden.

Angesichts der mit dem Stand der Technik verbundenen Nachteile besteht die Aufgabe der Erfindung in der Bereitstellung von Implantaten, welche auch in kleinlumige intrakranielle Gefäße durch herkömmliche Mikrokatheter hindurch eingebracht werden können, gut positionierbar und repositionierbar sind, bei Bedarf in den Mikrokatheter zurückgeführt werden können, und geeignet sind, Gefäßaussackungen und Fisteln so zu überbrücken, daß diese mit Okklusionsmitteln gefüllt werden können. Zudem wäre es wünschenswert, über Implantate zu verfügen, die sich dem Gefäßkaliber relativ frei anpassen können, d. h. nicht auf ein besonderes Gefäßkaliber hin gefertigt sind.

Diese Aufgabe wird erfindungsgemäß durch ein medizinisches Implantat der eingangs genannten Art gelöst, welches die Form eines längs offenen Schlauches mit zu einer Maschenstruktur untereinander verbundenen Stegen oder Filamenten aufweist, welche einseitig zu einer sich verjüngenden Struktur in einem Verbindungspunkt zusammenlaufen.

Anders ausgedrückt besteht das erfindungsgemäße Implantat aus einem flächigen Gegenstand, der aufgrund der ihm aufgeprägten übergeordneten Struktur die Form eines aufgeschlitzten Rohres oder Schlauches einnimmt, wobei sich die freien Schenkel vorzugsweise überlappen. In seiner volumenreduzierten Form liegt er weiter eingerollt vor, d. h. der Durchmesser des Implantates in seiner volumenreduzierten Form ist gegenüber dem der übergeordneten Struktur deutlich verringert. Nach Freisetzung des Implantates trachtet dieses, die ihm aufgeprägte Struktur einzunehmen und weitet sich aus, soweit es das das Implantat umgebende Gefäß zulässt. Diese Aufweitung nach Art einer sich aufweitenden Spiralfeder führt zu einer automatischen Anpassung des Implantates an das Gefäßkaliber bzw. -lumen dergestalt, daß es in Gefäßen unterschiedlichen Kalibers eingesetzt werden kann. Bei engen Gefäßen ergibt sich so eine relativ starke Überdeckung der beiden freien Schenkel, bei weiten Gefäßen eine geringe Überdeckung bzw. ein freibleibender Spalt, der gegebenenfalls, bei Gefäßverzweigungen, sogar gewünscht sein kann.

Das Implantat selbst hat eine Maschenstruktur aus untereinander verbundenen Stegen oder Filamenten. Stege treten auf, wenn das Implantat geschnittene Strukturen aufweist, wie sie beispielsweise bei Coronarstents häufig verwandt werden, eine Maschenstruktur aus Filamenten dann, wenn die Implantate als Matten mit gewirkter oder geflochtener Struktur oder aus miteinander verschweißten Einzelfilamenten vorliegen.

Wesentlich für die Erfindung ist, daß das Implantat eine Flächenstruktur ist, die zu einem längs offenen schlauchförmigen Gebilde gerollt ist, das sich an die Wandung des damit zu beaufschlagenden Gefäßes eng anlegen kann.

Die einseitig zu einer sich verjüngenden Struktur in einen Verbindungspunkt zusammenlaufenden Stege oder Filamente erlauben es, das noch mit einem Führungsdraht verbundene Implantat im Falle einer Fehlplatzierung oder unzureichenden Anpassung an den Implantationsort problemlos in den Katheter zurückzuziehen, um es gegen ein anderes Implantat auszutauschen oder es nach Repositionierung des Katheters erneut zu implantieren. Durch die sich verjüngende Struktur rollt sich das Implantat bei Eintritt in den Mikrokatheter enger zusammen und nimmt erneut seine volumenreduzierte Form an, wobei die Zugkraft des Führungsdrahtes und die vom Katheterrand ausgeübten Kräfte zusammenwirken.

Im Katheter selbst liegt das Implantat in seiner volumenreduzierten Form, etwa wie aufgerollter Maschendraht vor. Unter der Einwirkung des Führungsdrahtes kommt bei Anwendung von Schubkräften eine axiale Kompression hinzu, bei Entspannung und Einnahme der übergeordneten Struktur gegebenenfalls eine geringe Längenkontraktion. Die bei dilatierbaren Stents beobachtete Längenkontraktion tritt bei den erfindungsgemäßen Stents allerdings nur unwesentlich auf.

Der Verbindungspunkt des medizinischen Implantates am Ende der verjüngten Struktur ist gleichzeitig der Anknüpfungspunkt an den Führungsdraht, entweder direkt oder über ein Verbindungselement. Im Falle einer geschnittenen oder Strickmetallfolie handelt es sich dabei um den Punkt, in dem die Stege des Implantates zusammenlaufen. Bei einer Maschenstruktur aus einzelnen Filamenten laufen dort wenigstens zwei Filamente zusammen und sind durch Verschweißen oder Verkrimpen miteinander verbunden.

In der Regel ist der Verbindungspunkt gleichzeitig ein Verbindungselement bzw. ein Teil desselben, welches nach der Ablösung des Implantates vom Führungsdraht am Implantat verbleibt. Es kann zweckmäßig sein, diesen Verbindungspunkt in einer Platinspirale anzuordnen oder über eine Platinspirale mit einem Verbindungselement zu verbinden, welche gleichzeitig bei der Positionierung als röntgendichter Marker dienen kann. Besonders bevorzugt sind elektrolytisch korrodierbare Verbindungselemente, wie sie beispielsweise in der DE 10010 840 A1 beschrieben sind. Derartige Verbindungselemente ermöglichen es, das Implantat nach seiner korrekten Positionierung durch kurzfristige Stromeinwirkung von beispielsweise 10 bis 60 s vom Führungsdraht abzulösen.

Wie bereits dargestellt, besteht das medizinische Implantat aus einem flächigen Gebilde, das sich aufgrund der ihm vorgegebenen übergeordneten Struktur zu einem Schlauch zusammenrollt. Vorzugsweise kommt es dabei zu einer wenigstens geringen Überdeckung der freien Schenkel des Implantates.

Das Implantat selbst kann aus einer Folie bestehen, die, beispielsweise durch Lasertechnik, mit den entsprechenden Stegmustem versehen ist. Die Stegbreite beträgt beispielsweise 0,05 bis 0,2 mm. Die Fertigungstechnik ist die gleiche, wie sie bei röhrenförmigen Coronarstents angewandt wird. Alternativ dazu können auch Streckmetallfolien eingesetzt werden, wobei die Stegbreite sich in der gleichen Größenordnung bewegen. Hierbei ist es bevorzugt, die Folie anschließend zu glätten, so daß sich alle Stege in einer Ebene befinden. Die Foliendicke liegt in der Regel Im Bereich von 0,02 bis 0,2 mm. Die größeren Folienstärken erlauben auch den Einsatz in anderen Bereichen, beispielsweise als Coronarstents oder in anderen Körperbereichen, etwa im Gallengang oder im Harnleiter.

Die Maschenweite liegt in der Regel im Bereich von 0,5 bis 4 mm und kann innerhalb eines Implantates variieren. Gleiches gilt auch für die Stegbreite. So ist es im allgemeinen bevorzugt, im Bereich der Verjüngung größere Maschenweiten und -längen und/oder größere Stegbreiten oder stärkere Filamente zu verwenden. Im Bereich der sich verjüngenden Struktur ist im allgemeinen keine besonders große Stützwirkung und Abdeckung der Gefäßwand erforderlich, andererseits eine erhöhte Zug- und Schubfestigkeit.

Bei der Verwendung von auf Filamenten aufbauenden Maschenstrukturen können gewirkte oder gestrickte Strukturen verwendet werden wie auch durch Verschweißung verbundene Filamente. Die Filamentstärken liegen im allgemeinen im Bereich von 0,01 bis 0,1 mm und vorzugsweise bei 0,02 bis 0,076 mm. Für die Maschenweiten gilt das bereits oben gesagte.

Soweit die Maschenstruktur aus miteinander verschweißten einzelnen Filamenten besteht, wird vorzugsweise eine Laserschweißtechnik angewandt. Bei einem Maschengeflecht einzelner Filamente werden an und für sich bekannte Flecht-, Wirk- oder Stricktechniken angewandt, wie sie beispielsweise aus der Maschendrahtfertigung oder der Textiltechnik bekannt sind. Dabei sind besonders bevorzugt Maschengeflechte, die eine Wirkstruktur aufweisen, die herstellungsbedingt zu eingerollten Rändern führt, weil auf diese Art und Weise die erforderliche übergeordnete Struktur über das Wirkverfahren erzeugt werden kann. Besonders bevorzugt ist eine dem Textilfachmann als "Fluse" bekannte Wirkstruktur.

Ein besonderer Vorteil der erfindungsgemäßen medizinischen Implantate gegenüber herkömmlichen aufweitbaren Stents besteht darin, daß bei der Anpassung an das zu beaufschlagende Gefäß keine Längenkontraktion mehr stattfindet. Die längs offene Struktur mit der vorgegebenen "Wicklung" hat keinerlei Einfluß auf die Längenausdehnung des Stents. Die Folienstrukturen selbst haben sich auch bei der Positionierung unter Schub- und Zugwirkung als ausgesprochen dimensionsstabil erwiesen. Entsprechendes gilt für die Wirkstruktur und die Maschenstruktur aus verschweißten einzelnen Filamenten.

Als Filamente können auch solche verwandt werden, die aus einem Geflecht von Einzellitzen bestehen, also ein Seil darstellen. Geflechte aus 12 bis 14 Litzen mit einer Gesamtstärke von 0,02 mm haben sich als brauchbar erwiesen.

Soweit den Implantaten die übergeordnete Struktur nicht aufgrund der Wirk-, Strick- oder Flechttechnik aufgegeben werden kann, ist der Einsatz von Materialien sinnvoll, die über Formgedächtniseigenschaften verfügen. Solche Legierungen sind beispielsweise Titan und Nickel enthaltende Legierungen, die unter der Bezeichnung Nitinol bekannt geworden sind, Eisenbasis- oder Kupferbasislegierungen. Formgedächtniseigenschaften können auf einer spannungsinduzierten martensitischen Transformation beruhen wie auch auf einer temperaturinduzierten martensitischen Transformation oder Kombinationen der beiden.

Als Materialien für die Filamente kommen insbesondere auch Platin, Platinlegierungen, Gold und nichtrostender Stahl in Frage. Im allgemeinen können alle Dauerimplantatwerkstoffe der Medizintechnik eingesetzt werden, die über die benötigten Eigenschaften verfügen.

Wie schon erwähnt, weisen die erfindungsgemäßen Implantate insbesondere auch röntgendichte Marker auf, die die Überwachung der Positionierung und Implantation erlauben. Als solche Marker können proximal, insbesondere am Verbindungspunkt der Stege bzw. Filamente angeordnete Spiralen dienen. Vorzugsweise befinden sich röntgendichte Marker auch am distalen Ende des Implantates, insbesondere in Form von in die Maschenstruktur eingearbeiteten oder an die Maschenstruktur angehängten Platin- oder Platin/Iridiumelementen. Insbesondere können die Maschen des erfindungsgemäßen Implantates distal mit einer Öse versehen sein oder zu einer Öse auslaufen, in der sich das Markerelement flächeneben angeordnet befindet.

Die Erfindung betrifft ferner die Kombination des vorstehend beschriebenen Implantates mit einem Führungsdraht, der das Implantat an seinem distalen Ende lösbar angeordnet aufweist. Die Lösbarkeit wird insbesondere durch ein durch Stromeinwirkung korrodierbares Element herbeigeführt, wie es aus dem Stand der Technik bekannt ist. Bei dem Führungsdraht handelt es sich um einen ansonsten üblichen Führungsdraht, der geeignet ist, sowohl das Implantat durch einen Katheter hindurch zum Einsatz zu schieben und auch im Falle der Fehlpositionierung in den Katheter hinein zurückzuziehen. Naturgemäß kann die Korrosionsstelle auch im Bereich des Führungsdrahtes selbst liegen oder auf einer an und für sich bekannten mechanischen oder thermischen Ablösetechnik beruhen.

Schließlich betrifft die Erfindung auch ein System zur Verwendung bei der Behandlung von Aneurysmen oder anderer vaskulärer Fehlbildungen, das über einen ersten Mikrokatheter verfügt, einen ersten Führungsdraht zur Platzierung des ersten Mikrokatheters, einen zweiten Führungsdraht, der dazu bestimmt ist, das Implantat durch den ersten Mikrokatheter zu transportieren und zu platzieren sowie das Implantat, welches lösbar am distalen Ende des zweiten Führungsdrahtes angeordnet ist. Die eingerollte Struktur des Implantates und die Kombination mit dem Führungsdraht ermöglicht es, nach Platzierung des ersten Mikrokatheters den ersten Führungsdraht zu entfernen und den zweiten Führungsdraht mit dem Implantat einzuführen und zu handhaben. Bisherige Implantate dieser Art wurden stets mit einer sogenannten Pusher-Technik gesetzt, bei denen die Zurückholung des Implantates in der Regel ausgeschlossen war.

Gemäß einer bevorzugten Ausführungsform weist das System zusätzlich einen zweiten Mikrokatheter auf, der dazu ausgelegt und bestimmt ist, den zweiten Führungsdraht mit dem Implantat in sich verschiebbar aufzunehmen und durch den ersten Mikrokatheter zum Einsatz zu führen. Die Gleitfähigkeit fördernde Beschichtungen des zweiten Mikrokatheters können die Handhabung erleichtern.

Der erste Mikrokatheter ist ein an und für sich üblicher Mikrokatheter, wie er beispielsweise mit einem Durchmesser/Kaliber von 0,51 mm (20 mil) bis 0,36 mm (14 mil) in der Neuradiologie weit verbreitet ist.

Das System kann ferner übliche elektrische Einrichtungen zur elektrolytischen Ablösung des Implantats vom Führungsdraht an einer dafür vorgesehenen Ablösestelle aufweisen.

Die Erfindung wird durch die nachfolgend beschriebenen Abbildungen näher beschrieben. Es zeigen:
- Fig. 1:: Ein erfindungsgemäßes Implantat mit einer Wabenstruktur;
- Fig. 2:: eine weitere Ausführungsform eines erfindungsgemäßen Stents mit einer Wabenstruktur;
- Fig. 3:: eine dritte Ausführungsform eines erfindungsgemäßen Stents mit einer Wabenstruktur;
- Fig. 4:: eine Wirkstruktur, wie sie für erfindungsgemäße Implantate in Frage kommt;
- Fig. 5:: einen erfindungsgemäßen Stent samt Führungsdraht und Katheter;
- Fig. 6:: schematisch ein erfindungsgemäßes Implantat in seiner übergeordneten und in seiner volumenreduzierten Form;
- Fig.7:: ein Markerelement, wie es erfindungsgemäß zum Einsatz kommen kann;
- Fig. 8:: schematisch zwei Ablösestellen, über die das erfindungsgemäße implantat lösbar mit einem Führungsdraht verbunden werden kann.

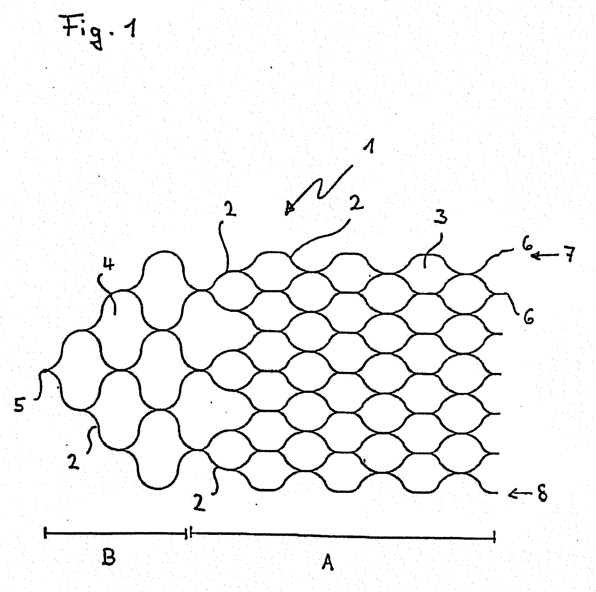

Das Implantat gemäß Figur 1 besteht aus einer Maschen- oder Wabenstruktur, die im vorliegenden Fall aus einer Mehrzahl von Filamenten besteht, die mit Hilfe von Laserschweißtechnik miteinander verbunden wurden. Das Implantat gliedert sich in einen eigentlichen funktionellen Teil A und die sich verjüngende proximale Struktur B, die sich u. a. durch eine unterschiedliche Maschengröße unterscheiden. Zur Wahrnehmung ihrer Rückhaltefunktion sind im funktionellen Teil A die Maschen 3 relativ eng, so daß sie geeignet sind, in einem Aneurysma angeordnete Occlusionsspiralen zurückzuhalten. Im sich verjüngenden proximalen Teil B des Implantats herrscht eine weite Maschenstruktur 4 vor, die auf eine minimale Occlusionswirkung hin optimiert wurde. Im Bereich der sich verjüngenden Struktur 2 weisen die Filamente vorzugsweise eine größere Stärke auf, um die im Verbindungspunkt 5 ansetzenden Schub- und Zugkräfte des Führungsdrahts beim Setzen des Implantats auf den funktionellen Teil A besser übertragen zu können. Filamentstärken im funktionellen Teil A liegen im allgemeinen in einer Größenordnung von 0,02 bis 0,076 mm im Teil B bei 0,076 mm und darüber.

Der proximale Teil B bildet im Verbindungspunkt 5 vorzugsweise einen Winkel von 45° bis 120° aus, insbesondere etwa 90°. Die Filamentstärke (bzw. Stegbreite) wie auch die Maschengröße und -form kann in weiten Bereichen variieren, je nach Anforderung an Stabilität, Flexibilität und dergleichen. Es versteht sich, daß sich auch der proximale Teil an die Gefäßwandung anlehnt und den Blutfluß im Gefäß nicht behindert.

Am distalen Ende laufen die Filamente 2 zu einer Reihe von "Schwänzen" 6 aus, die geeignet sind, Platinmarker zu tragen, die die Positionierung des Implantats erleichtern.

In seiner übergeordneten Struktur ist das Implantat 1 so aufgerollt, daß die Ränder 7 und 8 einander zumindest nahe sind, vorzugsweise sogar im Randbereich überlappen. In der volumenreduzierten Form ist das Implantat 2 nach Form einer Maschendrahtrolle soweit aufgerollt, daß die gebildete Rolle problemlos in einen Mikrokatheter eingeschoben und darin bewegt werden kann. Nach Freisetzung aus dem Mikrokatheter springt die aufgerollte Struktur auf und trachtet die ihm aufgeprägte übergeordnete Struktur einzunehmen und legt sich dabei an die Innenwand des zu beaufschlagenden Gefäßes eng an, wobei eine dort vorhandene Fistel, Gefäßabzweigung oder ein Aneurysma oberflächlich abgedeckt werden. Der Grad des "Aufrollens" wird dabei vom Gefäßvolumen bestimmt; in engeren Gefäßen kommt es zu einer stärkeren Überdeckung der Ränder 7 und 8 des Implantats 1, in weiten Gefäßen zu einer geringen Überdeckung oder sogar Unterdeckung, wobei darauf geachtet werden muß, daß das Implantat noch eine Restspannung aufweist.

Als Materialien kommen Legierungen mit Formgedächtniseigenschaften in Frage. Das fertige Produkt wird bei den für das Material üblichen Temperaturen getempert, so daß die aufgeprägte Struktur fixiert wird.

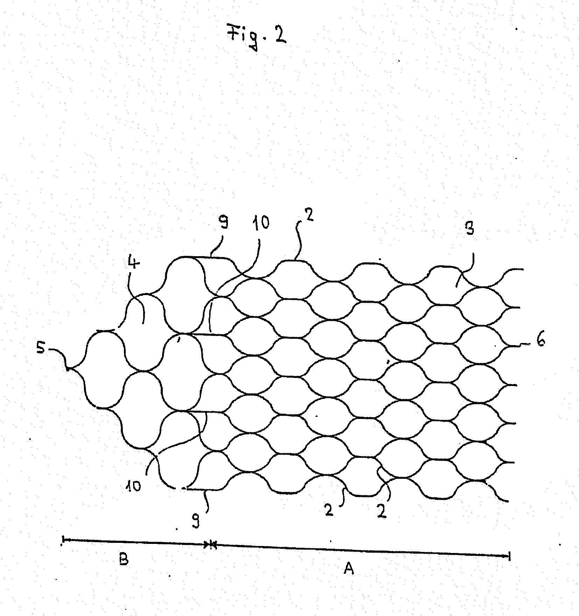

Figur 2 zeigt eine weitere Ausführungsform eines erfindungsgemäßen Stents 1 mit der bereits zuvor beschriebenen Wabenstruktur, bei der der sich verjüngende proximale Teil B durch zusätzliche Filamente 9 im peripheren Bereich 10 und im zentralen Bereich mit dem funktionellen Teil A verbunden ist. Die zusätzlichen Filamente 9 und 10 bewirken eine gleichmäßigere Übertragung von Zug- und Schubkräften von der proximalen Struktur B auf den funktionellen Teil A, so daß insbesondere bei einer gegebenenfalls notwendigen Repositionierung des Stents durch Zurückziehen in den Mikrokatheter die Zugkräfte besser übertragen werden können. Hierdurch erleichtert sich das erneute Einrollen des Stents. Gleichermaßen wird auch das Übertragen von Schubkräften beim Ausfahren und Setzen des Stents erleichtert und ein sanftes Absetzen ermöglicht. Im übrigen bezeichnen gleiche Ziffern gleiche Positionen.

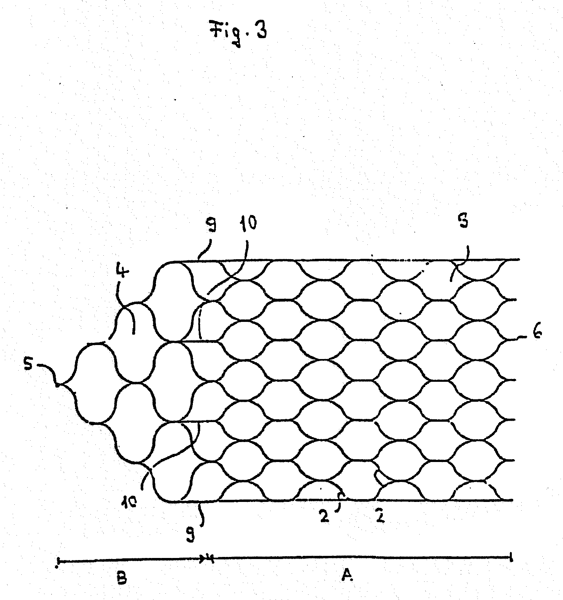

Figur 3 zeigt eine weitere Ausführungsform eines erfindungsgemäßen Stents 1 mit einer Wabenstruktur, bei der die Ränder 7 und 8 von im wesentlichen gradlinig verlaufenden Filamenten 9 gebildet werden. Gemäß dieser Ausführungsform wird der über den Punkt 5 vom Führungsdraht ausgeübte Schub oder Druck sehr direkt auf die Ränder 7 und 8 des funktionellen Stentteils A übertragen, was den in Bezug auf Figur 2 geschilderten Effekt weiter verstärkt.

Die Ausführungsform gemäß Figur 3 kann, wie auch diejenigen von Figur 1 und 2, auf einer geschnittenen Folie beruhen, d. h. die einzelnen Filamente 2, 9 und 10 sind durch einzelne Stege ersetzt, die bei einer mit Schneidtechnik bearbeiteten Folie stehen gelassen wurden. Laserschneidtechniken zur Herstellung von Stents mit einer Röhrenstruktur sind bekannt und vielfach beschrieben. Die Bearbeitung einer Folie zur Erzeugung eines für einen Stent geeigneten Musters verläuft entsprechend. Das Aufprägen der übergeordneten Struktur erfolgt auf gleiche Art und Weise, wie auch bei der Filamentausführung.

Mit Schneidtechnik bearbeitete Folien werden vorzugsweise elektrochemisch nachbearbeitet, um Grate und andere Unregelmäßigkeiten zu entfernen, eine oberflächliche Glättung herbeizuführen und Kanten zu runden. Derartige Bearbeitungsverfahren elektrochemischer Natur sind dem Fachmann bekannt und werden in großem Umfang in der Medizintechnik bereits eingesetzt. Im Zusammenhang ist anzumerken, daß die erfindungsgemäßen Stents, die auf zweidimensionalen Geometrien beruhen und denen eine dreidimensionale Struktur erst später aufgeprägt wird, grundsätzlich einfacher zu fertigen und zu bearbeiten sind, als die klassischen "Röhren"stents, die bereits bei der Fertigung in einer dreidimensionalen Struktur vorliegen und entsprechend aufwendiger Bearbeitungsverfahren/-vorrichtungen bedürfen.

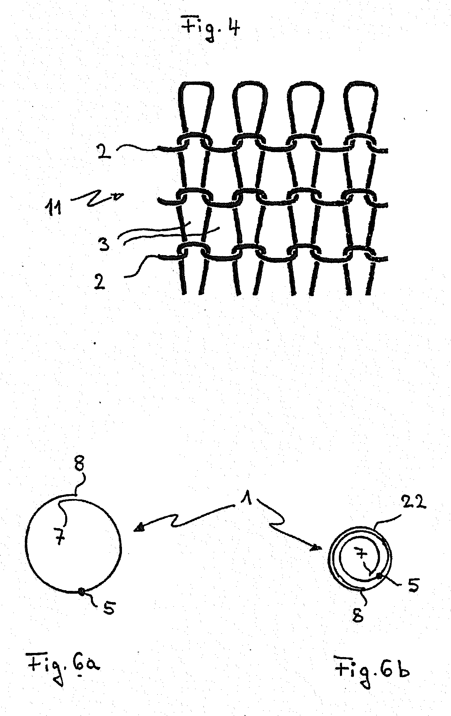

Wie zuvor dargelegt, kann die Maschenstruktur des erfindungsgemäßen Implantats aus einem Geflecht einzelner Filamente bestehen. Eine derartige Wirkstruktur ist in Figur 4 dargestellt, in der die einzelnen Filamente 2 nach Art eines rechts links Gestrickes zu einzelnen Maschen 3 einer Maschenstruktur 11 verwirkt sind. Derartige rechts links Gestricke werden auf bekannte Weise aus einer Nadelreihe hergestellt. Die rechts links Ware hat zwei verschieden aussehende Warenseiten, der rechten und der linken Maschenseite. Ein rechts links Gestrick ist in Querrichtung wenig elastisch und sehr leicht.

Besonders vorteilhaft ist, daß die Warenränder einer solchen Wirkstruktur einrollen, wie dies beispielsweise bei der "Fluse" der Fall ist, was für die hier verlangte übergeordnete Struktur und den Einsatzzweck von Vorteil ist. In diesem Fall kann die übergeordnete Struktur durch das Wirkverfahren aufgeprägt werden. Alternativ und zusätzlich ist aber auch hier der Einsatz von Formgedächtnislegierungen möglich und sinnvoll.

Für die Herstellung derartiger gewirkter Strukturen können bekannte Wirkverfahren eingesetzt werden. Da die erfindungsgemäßen Implantate von ihren Ausmaßen her allerdings außerordentlich klein sind - beispielsweise mit einer Größe von 2 x 1 cm - hat es sich als vorteilhaft erwiesen, die Implantate im Rahmen eines herkömmlichen Gewirkes oder Gestrickes aus textilen, nicht metallischen Filamenten herzustellen, beispielsweise in Form einer aus den jeweiligen metallischen Filamenten gefertigten Randes, der entweder angearbeitet ist oder von dem das Gestrick oder Gewirke ausgeht. Die Anordnung des metallischen Teils des Gestrickes oder Gewirkes am Rand ist von Bedeutung, um den Einrolleffekt zu erzielen. Die nicht metallischen textilen Teile der Wirkstruktur werden dann anschließend durch Veraschen, chemisches Zerstören oder Auflösen in einem geeigneten Lösemittel entfernt.

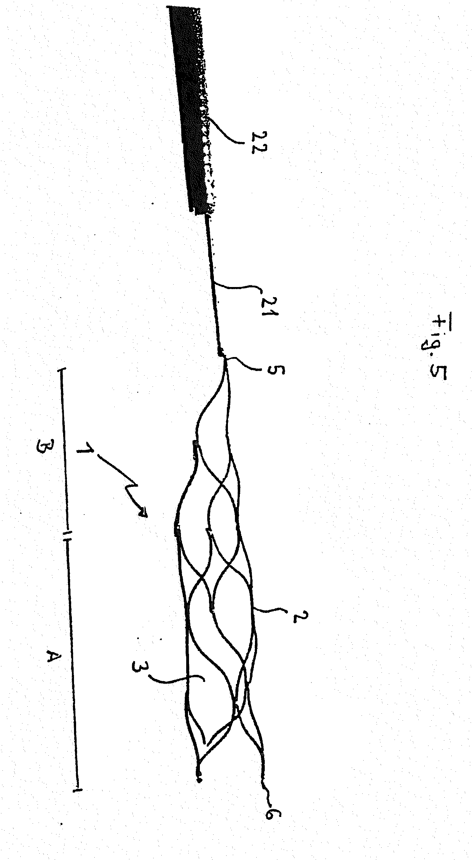

Figur 5 zeigt eine Kombination eines Führungsdrahts 21 mit daran festgelegtem Implantat 1, daß aus einzelnen miteinander verschweißten Filamenten 2 besteht. Deutlich zu erkennen sind die distalen Enden 6 und der Verbindungspunkt 5, an dem die Filamente des Implantats in der sich verjüngenden Struktur zusammenlaufen und der gleichzeitig die Verbindung zum Führungsdraht 21 herstellt. Der Führungsdraht 21 wird in einen Mikrokatheter 22 geführt, bei dem es sich um ein herkömmliches Fabrikat handelt.

Durch Verschieben des Führungsdrahts 21 im Katheter 22 wird das Implantat 1 herausgeschoben bzw. hineingezogen. Beim Hinausschieben aus dem Mikrokatheter trachtet die Maschenstruktur danach, die ihr aufgegebene übergeordnete Struktur einzunehmen, beim Hineinziehen rollt, bzw. faltet sich die Maschenstruktur entsprechend des räumlichen Gegebenheiten im Mikrokatheter wieder zusammen.

Aufgrund der Steifigkeit der Maschenstruktur kann das Implantat über den Führungsdraht 21 nahezu beliebig hin und her bewegt werden, bis es seine optimale Anordnung im Gefäßsystem gefunden hat.

Wie schon dargelegt, können herkömmliche Mikrokatheter eingesetzt werden. Der Vorteil des erfindungsgemäßen Implantats und der erfindungsgemäßen Kombination aus Implantat und Führungsdraht besteht allerdings darin, daß nach Setzen des Mikrokatheters mit einem üblichen Führungsdraht/Markersystem die erfindungsgemäße Kombination aus Führungsdraht 21 und Implantat 1 in den Mikrokatheter eingeschoben, durch diesen hindurch an den Einsatzort verbracht und dort ausgebracht werden kann. Alternativ ist es möglich, Führungsdraht 21 und Implantat 1 in einem zweiten Mikrokatheter kleineren Kalibers unterzubringen und mit diesem zweiten Mikrokatheter innerhalb des ersten gesetzten Mikrokatheters an den Einsatzort zu verschieben. In jedem Fall ergibt sich die einfache Führung des Implantats in beide Richtungen.

Figur 6 zeigt schematisch ein erfindungsgemäßes Implantat in seiner übergeordneten und in seiner volumenreduzierten Form. In seiner expandierten Struktur gemäß Figur 6a bildet das Implantat 1 eine ringförmige Struktur mit leichter Überlappung der Ränder 7 und 8. Die Abbildung zeigt das Implantat 1 von seinem proximalen Ende her in der Draufsicht, mit dem Verbindungspunkt 5 in etwa gegenüberliegend zu den Rändern 7 und 8. Am Verbindungspunkt 5 schließt sich in der erfindungsgemäßen Kombination der Führungsdraht 21 an.

Figur 6a zeigt das gleiche Implantat in seiner volumenreduzierten Form, wie es sich beispielsweise eingerollt in einem Mikrokatheter anordnet. Im dargestellten Fall ergeben sich insgesamt zwei Wicklungen des eingerollten Implantats 1 mit dem Verbindungspunkt 5 an der proximalen Seite und den beiden Seitenrändern 7 und 8 als Anfangs- und Endpunkte der Rolle bzw. Spirale. Die Struktur wird durch den Mikrokatheter 22 in der volumenreduzierten Form gehalten; bei Herausschieben des Implantats 1 aus dem Mikrokatheter 22 springt dieses auf zur expandierten Form von Figur 6a, vergleichbar einer Spiralfeder.

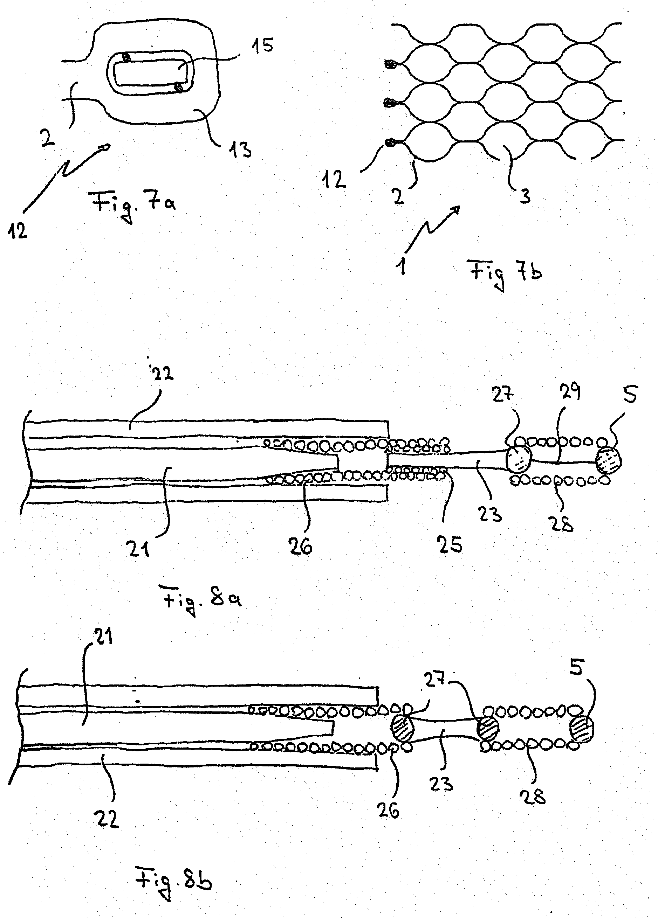

Figur 7a zeigt ein für die erfindungsgemäßen Implantate geeignetes Markerelement 12, wie es am distalen Ende des Implantats 1 zum Einsatz kommen kann. Das Markerelement 12 besteht aus einer "Öse" 13, die in ihrem Inneren flächeneben (in der Ebene des Implantats, ohne vorstehende Elemente) ein Markerplättchen 15 aus einem röntgendichten Material, beispielsweise Platin oder Platin-Iridium aufweist. Das Markerplättchen 15 ist über Laserschweißtechniken mit der umgebenen Implantatstruktur verbunden.

Figur 7b gibt ein Beispiel für die Anordnung der Markerelemente 12 am distalen Ende des Implantats 1 (s. Position 6 in Figur 1).

Figur 8 zeigt schematisch zwei Varianten einer Ablösestelle 8a und 8b, über die das erfindungsgemäße Implantat lösbar mit einem Führungsdraht verbunden ist. In beiden Fällen besteht die Ablösestelle 23 aus einem hantelförmigen Element, daß sich unter dem Einfluß von Strom bei Kontakt mit einem Elektrolyten auflöst. Das hantelförmige Element 23 gemäß Figur 8a weist an seinem proximalen (führungsdrahtseitigen) Ende eine Spiralstruktur 25 auf, die mit einer Verstärkungsspirale 26 des Führungsdrahts zusammenwirkt. Am distalen Ende befindet sich ein kugelförmiges Element 27, daß über Laserschweißtechnik mit einer Platinspirale 28 verbunden ist, die ihrerseits mit dem Verbindungspunkt 5 am proximalen Ende des Implantats verbunden ist. Die Platinspirale 28 dient zugleich als proximaler röntgendichter Marker des Implantats 1.

Zur Verstärkung der Verbindung zwischen dem kugelförmigen Element 27 und dem Verbindungspunkt 5 kann ein Verstärkungsdraht 29 sinnvoll sein. Alternativ kann aber auch die Platinspirale 28 konstruktiv so ausgelegt sein, daß sie den auf sie ausgeübten Zug- und Schubkräften Stand hält.

Für das Ablöseelement 23 kommt insbesondere Stahl in Frage, der unter dem Einfluß von Strom in einem Elektrolyten korrosionsanfällig ist. Zur Beschleunigung der Korrosion und Verminderung der Ablösezeit ist eine strukturelle oder chemische Schwächung der Hantel sinnvoll, beispielsweise durch Anschleifen oder eine thermische Behandlung.

Im allgemeinen hat der für den Elektrolyten zugängliche Bereich der Hantel 23 eine Länge von 0,1 bis 0,5 mm, insbesondere 0,3 mm.

Die Spiralstruktur 25 ist über Schweißpunkte sowohl mit dem hantelförmige Element 23 als auch mit der Verstärkungsspirale 26 des Führungsdrahts 21 verbunden. Der Führungsdraht 21 selbst ist im Mikrokatheter 22 verschiebbar gelagert.

Fig. 8b zeigt eine zweite Ausführungsform, die sich von der der Fig. 8a dadurch unterscheidet, daß das handeiförmige Element 23 an beiden Enden ein kugelförmiges Element 27 aufweist, welche ihrerseits distal mit dem Verbindungspunkt 5 des Implantats und proximal mit dem Führungsdraht 21 über Spiralen 28 bzw. 26 verbunden sind.

Es versteht sich, daß andere Ablöseprinzipien verwand werden können, etwa solche, die auf mechanischen Prinzipien beruhen oder auf dem Abschmelzen von Kunststoffverbindungen basieren.

## Patentansprüche

1. Stent mit einem proximalen und einem distalen Ende, welches zur Einnahme einer übergeordneten Struktur am Implantationsort vorgeformt ist, für die Einführung mittels eines Mikrokatheters (22) und eines am proximalen Ende angeordneten Führungsdrahts (21) jedoch in eine volumenreduzierte Form überführbar ist,
wobei der Stent (1) in seiner übergeordneten Struktur die Form eines längs offenen Schlauches annimmt und eine Wabenstruktur oder Maschenstruktur (3) untereinander verbundener Stege oder Filamente (2) aufweist, wobei der Stent einen funktionellen Teil (A) und an seinem proximalen Ende eine sich verjüngende Struktur (B) aufweist, in der die Stege oder Filamente (2) in einem Verbindungspunkt (5), der gleichzeitig ein Anknüpfungspunkt für den Führungsdraht (21) ist, zusammenlaufen,
**dadurch gekennzeichnet, dass**
sich der funktionelle Teil (A) und die sich verjüngende Struktur (B) zumindest in einer unterschiedlichen Wabengröße oder Maschengröße unterscheiden, wobei im funktionellen Teil (A) die Waben oder Maschen (3) relativ eng sind und wobei in der sich verjüngenden Struktur (B) eine relativ weite Wabenstruktur oder Maschenstruktur vorherrscht.

2. Stent (1) nach Anspruch 1, wobei die sich verjüngende Struktur (B) in einem röntgendichten Marker, bevorzugt einer als röntgendichter Marker ausgebildeten Platinspirale (28) zusammenläuft, insbesondere am Verbindungspunkt der Stege oder Filamente, wobei die Platinspirale aus Platin oder Platin-Iridium ausgebildet ist.

3. Stent (1) nach einem der vorhergehenden Ansprüche, der distal einen oder mehrere Marker (12), insbesondere Platinmarker, aufweist, die bevorzugt endständig an Knotenpunkten der Stege oder Filamente (2) angeordnet sind oder bevorzugt von einer Reihe von "Schwänzen" (6), zu denen die Stege oder Filamente (2) auslaufen, getragen werden, wobei die Maschen des Stents insbesondere distal mit einer Öse versehen sind oder zu einer Öse auslaufen, in der sich der Marker (12) flächeneben angeordnet befindet.

4. Stent (1) nach einem der vorhergehenden Ansprüche, wobei die sich verjüngende Struktur so ausgestaltet ist, dass sich der Stent durch die sich verjüngende Struktur bei Eintritt in den Mikrokatheter enger zusammenrollt und erneut seine volumenreduzierte Form annimmt, wobei eine Zugkraft des Führungsdrahtes und von einem Katheterrand ausgeübte Kräfte zusammenwirken.

5. Stent (1) nach einem der vorhergehenden Ansprüche, wobei der Stent aus einer Folie besteht, die, vorzugsweise durch Lasertechnik, mit entsprechenden Stegmustern versehen ist.

6. Stent (1) nach einem der vorhergehenden Ansprüche, wobei in der sich verjüngenden Struktur (B) eine größere Stegbreite oder größere Filamentbreite oder stärkere Filamente (2) oder stärkere Stege (9) vorherrschen.

7. Stent (1) nach einem der vorhergehenden Ansprüche, der zumindest teilweise aus einer Legierung mit Formgedächtniseigenschaften, insbesondere einer Titan und Nickel enthaltenden Legierung, die unter der Bezeichnung Nitinol bekannt geworden ist, besteht.

8. Stent (1) nach einem der vorhergehenden Ansprüche, wobei die sich verjüngende Struktur (B) durch zusätzliche Filamente oder Stege (9) in einem peripheren Bereich (10) und in einem zentralen Bereich mit dem funktionalen Teil (A) verbunden ist, so dass eine gleichmäßige Übertragung von Zug- und Schubkräften von der sich verjüngenden Struktur (B) auf den funktionalen Teil (A) bewirkt wird.

9. Stent (1) nach einem der vorhergehenden Ansprüche, wobei eine Maschenweite im Bereich von 0,5 mm bis 4 mm liegt.

10. Stent (1) nach einem der vorhergehenden Ansprüche, wobei in der sich verjüngenden Struktur (B) eine größere Maschenweite und -länge (4) vorherrscht als in dem funktionellen Teil (A).

11. Stent (1) nach einem der vorhergehenden Ansprüche, wobei eine Filamentstärke oder Stegbreite im funktionellen Teil (A) zwischen 0,02 mm und 0,076 mm liegt und wobei die Filamentstärke oder Stegbreite in der sich verjüngenden Struktur (B) bei 0,076 mm oder mehr liegt.

12. Kombination aus einem Stent (1) nach einem der vorhergehenden Ansprüche und einem Führungsdraht (21), der an dem Verbindungspunkt (5) direkt oder über ein Verbindungselement an den Stent (1) angeknüpft ist.

13. Kombination nach Anspruch 12, wobei der Führungsdraht (21) durch ein durch Stromeinwirkung korrodierbares Element an seinem distalen Ende an dem Stent (1) angeordnet ist.

14. Kombination nach Anspruch 12 oder 13, wobei der Führungsdraht (21) dazu geeignet ist, sowohl den Stent (1) durch einen Katheter zum Einsatz zu schieben als auch in den Katheter hinein zurückzuziehen.

15. Verfahren zur Herstellung eines Stents (1) nach einem der Ansprüche 1-11, wobei die Stege oder Filamente der Wabenstruktur bei einer mit Schneidtechnik bearbeiteten Folie stehen gelassen werden und anschließend die übergeordnete Struktur aufgeprägt wird.

## Claims

1. Stent with a proximal and a distal end, which is preformed to occupy an overarching structure at the implant site, for introduction by means of a microcatheter (22) and by means of a guide wire (21) arranged at the proximal end, which however can be converted into a shape of reduced volume,
wherein the stent (1) in its overarching structure adopts the shape of a longitudinally open flexible tube and has a honeycomb structure or mesh structure (3) of webs or filaments (2) joined to one another, wherein the stent has a functional part (A) and at its proximal end and has a tapering structure (B), in which the webs or filaments (2) converge in a connecting point (5), which at the same time is a contact point for the guide wire (21),
**characterised in that**
the functional part (A) and the tapering structure (B) differ at least in a different honeycomb size or mesh size, wherein in the functional part (A) the honeycombs or meshes (3) are relatively close together and wherein in the tapering structure (B) there is a relatively broad honeycomb structure or mesh structure.

2. Stent (1) according to Claim 1, wherein the tapering structure (B) converges in a radiopaque marker, preferably a platinum spiral (28) formed as a radiopaque marker, in particular at the connecting point of the webs or filaments, wherein the platinum spiral is formed from platinum or platinum-iridium.

3. Stent (1) according to any one of the preceding claims, which has at the distal end one or more markers (12), in particular platinum markers, which are preferably arranged terminally at nodal points of the webs or filaments (2) or are preferably carried by a row of "tails" (6), in which the webs or filaments (2) tail off, wherein the meshes of the stent are provided in particular distally with an eyelet or tail off in an eyelet, in which the marker (12) is arranged flat.

4. Stent (1) according to any one of the preceding claims, wherein the tapering structure is formed so that the stent is rolled up more tightly by the tapering structure on entry into the microcatheter and again assumes its volume-reduced shape, wherein a tensile force of the guide wire and forces exerted by a catheter edge cooperate.

5. Stent (1) according to any one of the preceding claims, wherein the stent consists of a foil, which is provided preferably by laser technology with corresponding web patterns.

6. Stent (1) according to any one of the preceding claims, wherein a larger web width or larger filament width or thicker filaments (2) or thicker webs (9) are present in the tapering structure (B).

7. Stent (1) according to any one of the preceding claims, which consists at least partially of an alloy with shape memory properties, in particular an alloy containing titanium and nickel, which has become known under the name Nitinol.

8. Stent (1) according to any one of the preceding claims, wherein the tapering structure (B) is joined by additional filaments or webs (9) in a peripheral region (10) and in a central region to the functional part (A), so that a uniform transmission of tensile forces and shear force from the tapering structure (B) to the functional part (A) is effected.

9. Stent (1) according to any one of the preceding claims, wherein the mesh width is in the range from 0.5 mm to 4 mm.

10. Stent (1) according to any one of the preceding claims, wherein in the tapering structure (B) there is a larger mesh width and length (4) than in the functional part (A).

11. Stent (1) according to any one of the preceding claims, wherein the filament thickness or web width in the functional part (A) is between 0.02 mm and 0.076 mm and wherein the filament thickness or web width in the tapering structure (B) is 0.076 mm or more.

12. Combination of a stent (1) according to any one of the preceding claims and a guide wire (21), which is coupled at the connecting point (5) directly or via a connecting element to the stent (1).

13. Combination according to Claim 12, wherein the guide wire (21) is arranged by an element corrodible under the action of current at its distal end on the stent (1).

14. Combination according to Claim 12 or 13, wherein the guide wire (21) is adapted to displace the stent (1) through a catheter for use and also to retract the stent into the catheter.

15. Method for producing a stent (1) according to any one of Claims 1 to 11, wherein in the case of a foil machined by a cutting technique the webs or filaments of the honeycomb structure are left and the overarching structure is then imposed.

## Revendications

1. Stent avec une extrémité proximale et une extrémité distale, lequel est préalablement formé pour prendre la forme d'une structure de niveau supérieur sur le lieu d'implantation et qui peut toutefois se rétrécir vers une forme à volume réduit pour l'introduction au moyen d'un micro-cathéter (22) et d'un fil de guidage (21) disposé au niveau de l'extrémité proximale,
le stent (1) prenant, dans sa structure de niveau supérieur, la forme d'un tuyau flexible ouvert dans le sens de la longueur et présentant une structure alvéolaire ou une structure à mailles (3) de nervures ou de filaments (2) reliés les uns aux autres, le stent présentant une partie fonctionnelle (A) et, au niveau de son extrémité proximale, une structure conique (B), dans laquelle les nervures ou les filaments (2) se réunissent en un point de liaison (5), qui est dans le même temps un point de rattachement pour le fil de guidage (21),
**caractérisé en ce que**
la partie fonctionnelle (A) et la structure conique (B) se distinguent au moins en une taille d'alvéoles ou de mailles différente, les alvéoles ou mailles (3) étant relativement étroites dans la partie fonctionnelle (A) et une structure alvéolaire ou structure à mailles relativement ouverte prédominant dans la structure conique (B).

2. Stent (1) selon la revendication 1, la structure conique (B) se rétrécissant en un marqueur opaque aux rayons X, de préférence en une spirale de platine (28) réalisée sous la forme d'un marqueur opaque aux rayons X, en particulier au niveau du point de liaison des nervures ou des filaments, la spirale de platine étant réalisée à partir de platine ou de platine-iridium.

3. Stent (1) selon l'une des revendications précédentes, lequel présente distalement un ou plusieurs marqueurs (12), en particulier un ou plusieurs marqueurs en platine, qui sont disposés de manière préférée, de manière terminale, au niveau de points nodaux des nervures ou des filaments (2) ou qui sont supportés de manière préférée par une rangée de « queues » (6) en lesquelles les nervures ou les filaments (2) se terminent, les mailles du stent étant pourvues, en particulier distalement, d'un oeillet ou se terminant en un oeillet, dans lequel se trouve le marqueur (12) disposé à plat.

4. Stent (1) selon l'une des revendications précédentes, la structure conique étant configurée de telle sorte que le stent s'enroule de manière plus étroite du fait de la structure conique lors de l'entrée dans le micro-cathéter et prend à nouveau sa forme à volume réduit, une force de traction du fil de guidage et des forces exercées par un bord de cathéter coopérant.

5. Stent (1) selon l'une des revendications précédentes, le stent étant constitué d'un film, qui est pourvu de motifs nervurés correspondants, de préférence par une technique laser.

6. Stent (1) selon l'une des revendications précédentes, une plus grande largeur de nervure ou une plus grande largeur de filament ou des filaments (2) plus épais ou des nervures (9) plus épaisses prédominant dans la structure conique (B).

7. Stent (1) selon l'une des revendications précédentes, qui est constitué au moins partiellement d'un alliage à mémoire de forme, en particulier d'un alliage contenant du titane et du nickel, qui s'est fait connaître sous le nom de Nitinol.

8. Stent (1) selon l'une des revendications précédentes, la structure conique (B) étant reliée à la partie fonctionnelle (A) par des filaments ou des nervures (9) supplémentaires dans une zone périphérique (10) et dans une zone centrale de sorte qu'une transmission homogène des forces de traction et de poussée de la structure conique (B) sur la partie fonctionnelle (A) soit entraînée.

9. Stent (1) selon l'une des revendications précédentes, une ouverture de mailles se situant dans la plage de 0,5 mm à 4 mm.

10. Stent (1) selon l'une des revendications précédentes, une ouverture et une longueur (4) de mailles étant plus grandes dans la structure conique (B) que dans la partie fonctionnelle (A).

11. Stent (1) selon l'une des revendications précédentes, une épaisseur de filament ou une largeur de nervure dans la partie fonctionnelle (A) se situant dans la plage comprise entre 0,02 mm et 0,076 mm et l'épaisseur de filament ou la largeur de nervure dans la structure conique (B) étant de l'ordre de 0,076 mm ou plus.

12. Combinaison composée d'un stent (1) selon l'une des revendications précédentes et d'un fil de guidage (21), qui est directement rattaché au niveau du point de liaison (5) ou qui est rattaché par l'intermédiaire d'un élément de liaison au niveau du stent (1).

13. Combinaison selon la revendication 12, le fil de guidage (21) étant disposé au niveau du stent (1) par un élément pouvant être corrodé sous l'action d'un courant au niveau de son extrémité distale.

14. Combinaison selon la revendication 12 ou 13, le fil de guidage (21) étant adapté à la fois pour pousser le stent (1) à travers un cathéter afin de l'insérer et pour le retirer à l'intérieur du cathéter.

15. Procédé servant à fabriquer un stent (1) selon l'une des revendications 1 - 11, les nervures ou les filaments de la structure alvéolaire étant laissés en place dans le cas d'un film usiné à l'aide d'une technique de découpage et la structure de niveau supérieur étant immédiatement après imprimée.
